# EUROPEAN PATENT APPLICATION

(11) **EP 4 470 554 A1**
(43) Date of publication of application: **04.12.2024**
(21) Application number: 22923674.0
(22) Date of filing: 30.12.2022
(51) Int. Cl.: A61K 38/48, A61P 9/08, A61P 9/10, A61P 25/28

(54) **USE OF KALLIKREIN I OR DERIVATIVE THEREOF IN TREATMENT OF VCI, PSCI OR CSVD**

(30) Priority: 28.01.2022 CN 202210106039
(71) Applicant: Zonhon Biopharma Institute, Inc., Changzhou, Jiangsu 213125 (CN)
(72) Inventor: MA, Bruce Yong, hangzhou, Jiangsu 213125 (CN); JIANG, Chenyang, hangzhou, Jiangsu 213125 (CN); WANG, Jun, hangzhou, Jiangsu 213125 (CN); WANG, He, hangzhou, Jiangsu 213125 (CN); XU, Zhen, hangzhou, Jiangsu 213125 (CN)
(74) Representative: Axe PI
(86) International application number: PCT/CN2022/144054
(87) International publication number: WO 2023/142889

(57) **Abstract**

The present invention relates to use of kallikrein I or derivative thereof in the treatment of vascular cognitive impairment (VCI), post-stroke cognitive impairment (PSCI) or cerebral small vessel disease (CSVD).

## Description

### TECHNICAL FIELD

The present invention relates to the use of kallikrein I or its derivatives in the treatment of vascular cognitive impairment (VCI), post-stroke cognitive impairment (PSCI), or cerebral small vessel disease (CSVD).

### BACKGROUND OF THE INVENTION

With the development of society and aging population, the incidence of cerebrovascular disease is increasing year by year, which seriously affects the quality of life of patients and their families. The concept of vascular cognitive impairment (VCI) was first proposed by Hachinski and Bowler in 1993. It refers to a large group of syndromes ranging from mild cognitive impairment to dementia caused by risk factors of cerebrovascular disease (such as hypertension, diabetes, hyperlipidemia, etc.), obvious cerebrovascular disease (such as cerebral infarction and cerebral hemorrhage, etc.), or less obvious cerebrovascular disease (such as leukoaraiosis, chronic cerebral ischemia). (Chinese Guidelines for the Diagnosis and Treatment of Vascular Cognitive Impairment (2019*)* , *"*Chinese Guidelines for theDiagnosis and Treatment of Vascular Cognitive Impairment " in Chinese Guidelines for the Prevention and Treatment of Stroke (2021*) )*

Patients with vascular cognitive impairment have cognitive impairment and psychoactive behavioral symptoms. The cognitive impairment of some patients mainly presents with the impairment of abstract thinking, concept formation and transformation, thinking flexibility, information processing speed and other functions, while memory is relatively preserved. Some patients present with multi-domain impairment and memory impairment. Drugs for cognitive impairment in VCI include cholinesterase inhibitors and non-competitive N-methyl-D-asparagine receptor antagonists. Psychoactive behavioral symptoms include abnormal perception, thinking, mood, and behavior, such as apathy, depression, irritability, agitation/aggression, and drugs for the treatment include antidepressant drugs such as serotonin reuptake inhibitors (*"Chinese* Guidelines for the Diagnosis and Treatment of Vascular Cognitive Impairment "). Post-stroke cognitive impairment (PSCI), cognitive impairment caused by cerebral small vessel disease (CSVD) and Alzheimer's disease (AD) with vascular lesions are important subtypes of VCI.

Post-stroke cognitive impairment emphasizes that stroke triggers cognitive dysfunction, which also involves cognitive impairment and psychoactive behavioral symptoms. Considering that there is some overlap in neuropathological and neurobiochemical mechanisms among PSCI, VCI and AD, the Chinese Expert Consensus on the Management of Post-Stroke Cognitive Impairment (2021 *)* recommends to refer to relevant research and evidence of VCI and AD. The drugs for the treatment of cognitive impairment generally include acetylcholinesterase inhibitors and non-competitive N-methyl-D-asparagine receptor antagonists (memantine). The drugs for the treatment of post-stroke depression generally include antidepressants. The latest *"*2021ESO/EAN Joint Guidelines on Post-stroke Cognitive Impairment" issued by the European Stroke Organization points out that there is no drug for the treatment of post-stroke cognitive impairment. Only one trial specifically focused on post-stroke cognitive impairment and had no positive results. The guideline also points out that there is a significant shortage of high-quality data from randomized controlled trials (RCTS) for PSCI.

Cerebral small vessel disease (CSVD) is a series of clinical, imaging and pathological syndromes caused by a variety of risk factors affecting small arteries, arterioles, capillaries, venules and venules in the brain. It is divided into acute CSVD and chronic CSVD. Acute CSVD can lead to ischemic stroke. At present, it is recommended to refer to the prevention and treatment of acute ischemic stroke, such as antihypertensive, thrombolysis, antiplatelet, and lipid-lowering therapy. Patients with chronic CSVD may have cognitive impairment, movement disorder, emotional disorder, defecation disorder and other symptoms. It is generally recommended that symptomatic treatment be carried out after a clear diagnosis. For cognitive impairment caused by CSVD, cholinesterase inhibitors and non-competitive N-methyl-D-asparagine receptor antagonists can be selected, and antidepressants such as serotonin reuptake inhibitors are generally selected for depression (" *Chinese Expert Consensus on the Diagnosis and Treatment of Cerebral Small Vessel Disease* ").

### SUMMARY OF THE INVENTION

The first technical problem addressed by this application is to provide a new drug for the treatment of vascular cognitive impairment. In particular, the use of kallikrein I or its derivatives in the preparation of drugs for the treatment or improvement of vascular cognitive impairment is provided.

The invention also provides a pharmaceutical composition, comprising kallikrein I or a derivative thereof for the treatment or improvement of vascular cognitive impairment.

The present invention also provides a method of treating or improving vascular cognitive impairment by administering kallikrein I or a derivative thereof to the patient.

Vascular cognitive impairment includes but is not limited to post-stroke cognitive impairment, cognitive impairment caused by cerebral small vessel disease, Alzheimer's disease with vascular lesion, etc.

Preferably, the vascular cognitive impairment is post-stroke cognitive impairment.

Preferably, the vascular cognitive impairment is cognitive impairment due to cerebral small vessel disease.

Preferably, the vascular cognitive impairment is Alzheimer's disease with vascular lesion.

The second technical problem addressed by this application is to provide a new drug for the treatment of cerebral small vessel disease. In particular, the use of kallikrein I or its derivatives in the preparation of drugs for the treatment or improvement of cerebral small vessel disease is provided.

The present application also provides a pharmaceutical composition, comprising kallikrein I or a derivative thereof for the treatment or improvement of cerebral small vessel disease.

The present application also provides a method of treating or improving cerebral small vessel disease by administering kallikrein I or a derivative thereof to the patient.

The kallikrein I may be naturally extracted kallikrein I or recombinant kallikrein I. The kallikrein I derivative may be a full-length protein of kallikrein I, a partial protein, a mutant, a fusion protein, or various forms of modified product.

Preferably, the kallikrein I derivative is polyethylene glycol modified kallikrein I.

The kallikrein I or its derivatives can be administered alone or in combination with other drugs that treat or improve cerebral small vessel disease and vascular cognitive impairment, such as cholinesterase inhibitors, non-competitive N-methyl-D-asparagine receptor antagonists, etc.

Administration methods include, but not limited to, injection and oral administration. The injection methods include but not limited to intravenous injection, subcutaneous injection, intramuscular injection, etc.

Well-recognized animal models of vascular cognitive impairment include: The 4-vessel occlusion (4-VO) method, modified 4-VO method, 3-stage 4-VO method, 2-vessel occlusion (2-VO) method, modified 2-VO, and modified common carotid artery occlusion (mCCAO) in rats, and bilateral CCA stenosis (BCAS) and asymmetric CCA surgery (ACAS) in mice (*Advances in Animal Models of Vascular Cognitive Impairment).*

The recognized animal models of cerebral small vessel disease mainly include bilateral common carotid artery ligation model, bilateral common carotid artery stenosis model (BCAS), and stroke-susceptible spontaneously hypertensive rat model, etc. (*"Chinese Expert Consensus on Translational Medical Research of Cerebral Small Vessel Disease* ").

As a more recognized experimental animal model in the study of vascular cognitive impairment and cerebral small vessel disease, the cerebral blood flow of BCAS model animals is significantly reduced, which causes chronic hypoperfusion injury, resulting in increased permeability of the blood-brain barrier and white matter damage, which in turn causes memory dysfunction and motor function impairment. The results of the present study showed that kallikrein I or its derivatives could significantly improve the impaired motor coordination function, impaired forelimb muscle strength, working memory, spatial learning and memory function in BCAS mice. Therefore, kallikrein I or its derivatives can be used to treat vascular cognitive impairment and cerebral small vessel disease.

In addition, post-stroke cognitive impairment, cognitive impairment caused by cerebral small vessel disease, and Alzheimer's disease with vascular lesions are important subtypes of vascular cognitive impairment. In the clinical treatment of these diseases, the same symptomatic treatment drugs are used. For cognitive impairment, the treatment guidelines or expert consensus for vascular cognitive impairment, post-stroke cognitive impairment, and cerebral small vessel disease generally recommend acetylcholinesterase inhibitors and non-competitive N-methyl-D-asparagine receptor antagonists (memantine). For psychoactive behavioral symptoms such as depression, antidepressants are generally recommended for the medical treatment. Therefore, kallikrein I or its derivatives can be used to treat various subtypes of vascular cognitive impairment, such as post-stroke cognitive impairment, cognitive impairment caused by cerebral small vessel disease, Alzheimer's disease with vascular lesions, etc.

The Kinin-kallikrein system (KKS) is involved in a variety of physiological and pathological processes, such as the regulation of cardiovascular, renal and nervous system functions. The KKS system includes kallikrein, kininogen, kinin, kinin receptors (B1 and B2 receptors) and kininase. Kallikrein is a serine protease. It can be divided into two categories: plasma kallikrein I(PK) and tissue kallikrein I(TK), which play important physiological roles. At present, it is believed that human tissue kallikrein is composed of at least 15 members (KLK1-KLK15), among which there are many studies on tissue kallikrein I(KLK1). KLK1 plays a series of biological roles by converting kininogens into kinins and acting on corresponding receptors. In China, two kinds of kallikrein I for injection have been marketed, and the indications include microcirculation disorders and mild to moderate acute ischemic stroke.

Vascular cognitive impairment and cerebral small vessel disease are quite different from the approved indications of kallikrein I. In terms of pathological mechanism, acute ischemic stroke is brain tissue ischemia, hypoxic necrosis due to local cerebral blood flow reduction or blood supply interruption caused by stenosis, occlusion or thrombosis of the main arteries or branch arteries of the cerebral on the basis of vascular wall lesions caused by various reasons. VCI refers to a large group of syndromes ranging from mild cognitive impairment to dementia caused by cerebrovascular risk factors (such as hypertension, diabetes, hyperlipidemia, etc.), obvious cerebrovascular diseases (such as cerebral infarction and cerebral hemorrhage, etc.) or less obvious cerebrovascular diseases (such as leukoaraiosis, chronic cerebral ischemia, etc.). The abnormal function of NVU (neurovascular unit) plays an important role in the pathogenesis of cerebral small vessel disease. Changes in the structure or function of NVU caused by any reasons can lead to CSVD. The common mechanisms include chronic cerebral ischemia and hypoperfusion, endothelial dysfunction, blood-brain barrier disruption, interstitial fluid reflux disorder, inflammatory response and genetic factors, etc. There is an interaction between different mechanisms. (*The Neurovaseular Unit Coming of Age: a Journey Through Neurovaseular Coupling in Health and Disease)*

In addition, the diagnosis and treatment of acute ischemic stroke, vascular cognitive impairment and cerebral small vessel disease are quite different. The most effective treatment for acute ischemic stroke is vascular recanalization therapy within the time window, including intravenous thrombolysis, mechanical thrombectomy, angioplasty, etc. The success rate of treatment is closely related to disease course. Drug therapy includes antiplatelet, anticoagulation, defibrination, volume expansion, vasodilatation, statins, neuroprotective drugs, etc. Cognitive impairment and psychoactive behavioral symptoms are common in VCI and PSCI. Drugs for cognitive impairment in VCI and PSCI include cholinesterase inhibitors, non-competitive N-methyl-D-asparagine receptor antagonists. Drugs for psychoactive behavioral symptoms are as antidepressant drugs such as serotonin reuptake inhibitors. Patients with chronic cerebral small vessel disease may have symptoms such as cognitive impairment, movement disorders, emotional disorders, and defecation disorders. It is generally recommended that symptomatic treatment be carried out after a definite diagnosis. For cognitive impairment caused by CSVD, cholinesterase inhibitors and non-competitive N-methyl-D-asparagine receptor antagonists can be selected. For depression, antidepressants such as 5-hydroxytryptamine reuptake inhibitors are generally selected.

The application found that kallikrein I or its derivatives can be used to treat vascular cognitive impairment, post-stroke cognitive impairment, cerebral small vessel disease, etc, which is different from the approved indications of kallikrein I. It provides a new method for the treatment of vascular cognitive impairment, post-stroke cognitive impairment and cerebral small vessel disease.

### BRIEF DESCRIPTION OF THE DRAWINGS

FIG. 1: KLK1 and its mutants activated phosphorylation of downstream CREB proteins.
FIG. 2: Lys-BK activated phosphorylation of downstream ERK1/2 and CREB proteins.
FIG. 3: Effect of drugs on latency to fall in rotarod test (Day 13) of experiment 1.
FIG. 4: Effect of drugs on latency to fall in rotarod test (Day 26) of experiment 1.
FIG. 5: Effect of drugs on latency to fall in wire hang test (Day 20) of experiment 1.
FIG. 6: Effect of drugs on latency to fall in wire hang test (Day 28) of experiment 1.
FIG. 7: Effect of drugs on alternation behavior in Y Maze test (Day 29) of experiment 1.
FIG. 8: Effect of drugs on alternation behavior in Y Maze test (Day 44) of experiment 1.
FIG. 9: Effect of drugs on platform latency in Morris maze test (Day 48) of experiment 1.
FIG. 10: Effect ofdrugs on latency to fall in rotarod test of experiment 2.
FIG. 11: Effect of drugs on latency to fall in wire hang test of experiment 2.
FIG. 12: Effect of drugs on platform latency (distance traveled in quadrant III) in morris maze test of experiment 2.
FIG. 13: Effect of drugs on platform latency (time spent in quadrant III) in morris maze test of experiment 2.

### DETAILED DESCRIPTION OF THE INVENTION

Unless otherwise specified, the technical terms or abbreviations of this application have the following meanings:
Kallikrein I: Specifically tissue kallikrein I, KLK1.
hKLK1: unmutated human tissue kallikrein I with sequence homology to
native human tissue kallikrein I; It covers various homologs of human KLK1, including but not limited to KLK1 as shown in Genbank accession numbers AAA59455.1, NP002248.1, AAA36136.1, AAP35917, AAU12569, etc. In a specific embodiment, the sequence was shown as SEQ ID NO: 5.

Three glycosylation sites N78, N84 and N141 of KLK1: asparagine at position 78, 84 and 141 of KLK1 amino acid sequence, respectively. The corresponding N-glycosylation motifs are NMS, NHT, and NFS, with N for asparagine, M for methionine, S for serine, H for histidine, T for threonine, and F for phenylalanine.

High glycosylated hKLK1: unmutated hKLK1, with high glycosylation, that is, there are three glycosylation sites N78, N84 and N141 in high glycosylated hKLK1, each N-glycosylation are glycosylated.

Low glycosylated hKLK1: unmutated hKLK1 with low glycosylation, that is, there are three glycosylation sites N78, N84 and N141 in low glycosylated hKLK1, but no glycosylation, or only a small amount of glycosylation at the site N141. Recombinant high glycosylated hKLK1, low glycosylated hKLK1 can be isolated by conventional purification methods, such as hydrophobic chromatography, anion chromatography, cation chromatography, or a combination thereof. The applicant unexpectedly found that the activity of low glycosylated KLK1 was much higher than that of high glycosylated KLK1.

PEG-hKLK 1 (high glycosylated): polyethylene glycol modified high glycosylated hKLK1, said high glycosylated hKLK1 refers to the above unmutated hKLK1 with high glycosylation.

PEG-hKLK1(low glycosylated): polyethylene glycol modified low glycosylated hKLK1, said low glycosylated hKLK1 refers to the above unmutated hKLK1 with low glycosylation.

hKLK1X: hKLK1 mutant. KLK1 is an enzyme with rich glycosylations. N-glycosylation is the most concerned glycosylation in glycoprotein drugs, where the carbohydrate chain is linked to the free -NH2 group of a specific asparagine in the peptide chain. The N-glycosylation motif must be NXS or NXT, where N represents asparagine, S represents serine, T represents threonine, X represents other amino acids except proline. And asparagine that does not meet the above conditions can not be N-glycosylated. hKLK1X1, hKLK1X2, hKLK1X3, and hKLK1X4 represent different mutants, the sequences are shown as SEQ ID NO: 1,2,3,4. Mutation was on the glycosylation site N141, which asparagine was mutated to four different types of amino acids, namely neutral polar amino acid glutamine (Gln), acidic amino acid aspartic acid (Asp), basic amino acid arginine (Arg), and aliphatic amino acid alanine (Ala). The mutant had no NFS sequence and had only two glycosylation sites, resulting in a more homogeneous product and a higher yield of low glycosylated hKLK1. Surprisingly, the activity of the low glycosylated KLK1 mutant was much higher than that of the unmutated low glycosylated KLK1.

The embodiments were provided for illustrative purposes only and not to limit the scope of this application. All the following schemes can change the original NFS sequence so that it did not constitute an N-glycosylation motif, and there was no glycosylation at N141. Thus, the product was more homogeneous and the yield of low glycosylated hKLK1 was higher. For example N at NFS (i.e., N141) was mutated, 0, 1 or 2 amino acids of F and S were mutated to any other amino acid. Or F(F142) at NFS was mutated to proline, and 0, 1, or 2 amino acids of N and S were mutated to any other amino acid. Or S (i.e. S 143) at NFS was mutated to any amino acid except threonine, and 0, 1, or 2 amino acids of N and F were mutated to any other amino acid. Surprisingly, the activity of mutant KLK1 was much higher than that of unmutated KLK1.

hKLK1X: hKLK1 mutant. hKLK1X1, hKLK1X2, hKLK1X3, and hKLK1X4 represent different mutants.

PEG-hKLK1X: Polyethylene glycol modified hKLK1 mutant.

KLK1 derivatives: It includes not only the full-length protein, but also partial KLK1 protein; not only the KLK1 mutant described in this application, but also the protein obtained by further mutation,, fusion protein (including but not limited to albumin fusion, Fc fusion, etc.) and various forms of modification (including but not limited to pegylated modification) on the basis of the KLK1 mutant described in this application.

Polyethylene glycol: PEG, usually formed by the polymerization of ethylene oxide, has branched, linear, and multi-arm forms. In general, those with molecular weights below 20,000 are referred to as PEG and those with larger molecular weights are referred to as PEO. Ordinary polyethylene glycol has a hydroxyl group at each end. If one end is blocked with a methyl group, methoxy polyethylene glycol (mPEG) is obtained.

Polyethylene glycol modifiers: PEG modifiers refer to PEG derivatives with functional groups, which are activated polyethylene glycol and can be used for protein and peptide drug modification. The polyethylene glycol modifier used in this application was purchased from ZonHon Biopharma Institute,INC. or Beijing Jiankai Technology Co., LTD. The actual molecular weight of PEG modifier can be 90%-110% of the labeled value. For example,such as molecular weight of PEG5K may be 4.5-5.5 kDa.

PEG5K used specifically refers to M-SPA-5K, a straight chain monomethoxy polyethylene glycol succinimidyl propionate with a molecular weight of approximately 5kDa, with structure as shown in formula (1), with n being an integer from 105 to 110,

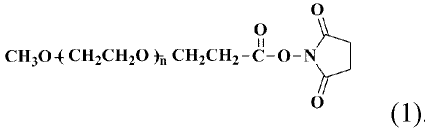

PEG10K used specifically refers to M-SPA-10K, a straight chain monomethoxy polyethylene glycol succinimidyl propionate with a molecular weight of about 10 kDa, with structure as shown in formula (1), with n being an integer from 220 to 225.

PEG30K used specifically refers to Y-PALD-30K, which is a branched polyethylene glycol propionaldehyde with molecular weight of about 30kD, with structure as shown in formula (2), m being an integer from 335 to 340,

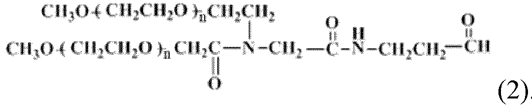

PEG40K used specifically refers to Y-PALD-40K, a branched polyethylene glycol propionaldehyde with molecular weight of about 40kD, with structure as shown in formula (2), m being an integer of 450 to 455.

PEG modifiers suitable for KLK1 are not limited to the PEG modifiers used in specific embodiments, but other commonly known PEG modifiers may also be used.

The pegylated KLK1 in the following embodiments was PEGylated and purified using a method well known in the art. For example, it can be prepared by referring to Chinese patent CN109498815A, CN107760661A or CN202111353294.2.

### Example 1 In vitro activity detection of recombinant human kallikrein I(hKLK1) and its mutant (hKLK1X)

### 1. In vitro activity evaluation based on artificial substrates

KLK1 exerts its biological function in vivo through catalyzing the hydrolysis of LMWK to release lysyl bradykinin, which involves the cleavage of the carboxy-terminal peptide bond of arginine (Arg). P-nitroaniline (PNA) was generated by hydrolyzating the amide bond between Arg and p-nitroaniline in the synthetic chromogenic substrate S-2266(H-D-Val-Leu-Arg-PNA). Therefore, PNA was detected at 405nm to evaluate the in vitro biological activity of recombinant hKLK1 and its mutants. One unit IU of activity was defined as the amount of enzyme that hydrolyzed 1µmol S-2266 to PNA per minute at 37°C and pH8.0. The reaction system consisted of 200µl 20mM trometamol buffer, 10µl test article, and 20µl 20mM S-2266 substrate solution, which were placed in water bath at 37°C for accurate reaction for 10min. The reaction was terminated by adding 20µl 50% acetic acid solution. The amount of PNA produced in the reaction system was quantified based on a standard curve fitted with different concentrations of PNA standards. The in vitro biological activities of hKLK1, hKLK1 mutants and hKLK1-PEG modified samples were detected by the above method.

As shown in the table below, the activity of mutant samples (hKLK1X1, hKLK1X2, hKLK1X3, hKLK1X4) was higher than that of unmutated low glycosylated hKLK1 samples.

**Table 1**

| **Sample** | **Specific activity (IU/mg)** | **Relative activity** |
|---|---|---|
| low glycosylatedhKLK1 | 5.1 | 100% |
| hKLK1X1 | 7.0 | 137.3% |
| hKLK1X2 | 7.1 | 139.2% |
| hKLK1X3 | 5.7 | 111.8% |
| hKLK1X4 | 5.7 | 111.8% |
| urinary kallikrein (Guangdong Tianpu) | 4.5 | 88.2% |

### 2. In vitro activity evaluation based on natural substrates

### Enzymatic reaction and liquid phase detection

KLK1 exerts its biological function in vivo through catalyzing the hydrolysis of LMWK to release lysyl bradykinin. This example compared the enzymatic reaction to produce the effector molecule bradykinin at different ratios of substrate (LMWK) to enzyme (KLK1 or its PEG modified KLK1). The produced effector molecules were separated by reverse phase chromatography, the peak area of the product is calculated, and the curve of bradykinin production with different ratios of substrate to enzyme was drawn. Compare the amount of effector molecules produced by different test samples under the same reaction methods. Through this in vitro method simulating the in vivo effect, the in vivo effects of different tested samples were indirectly compared.

Kb was KLK1 extracted from porcine pancreas. PEG-Kb was PEG10K-modified Kb. PEG10K-hKLK1X1 and PEG-Kb can be prepared according to the conventional method of polyethylene glycol modified drugs.

The samples were mixed according to the following table (in PEG modified samples, the molar mass of the modified active substance was converted). The mixed samples were placed in a 37°C thermostat, incubated for 15min, accurately timed, and the reaction was terminated by adding 50% acetic acid solution according to the volume ratio of 10:1.

**Table 2**

| **System (substrate: enzyme mol/mol)** | **Substrate µl** | **Enzyme µl** | **50% acetic acid µl** | **Total volume of system µl** |
|---|---|---|---|---|
| 1: 1 | 10 | 10 | 2 | 22 |
| 5: 1 | 50 | 10 | 6 | 66 |
| 10: 1 | 50 | 5 | 5.5 | 60.5 |
| 15: 1 | 60 | 4 | 6.4 | 70.4 |
| 20: 1 | 60 | 3 | 6.3 | 69.3 |

After the termination of the reaction, the sample was placed in a desktop centrifuge, centrifuged at 12000rpm for 5min, and the supernatant was taken. Detect with Waters ACQUITY UPLC H-Class, the mobile phase A was 0.1% TFA-H₂O, the mobile phase B was 0.1% TFA-ACN, the detection wavelength was 214nm, the column temperature was 30°C, the sample loading volume was 10µl, the flow rate was 0.2ml/min, the running time was 35min, and the running gradient was as follows:

**Table 3**

| **Run time min** | **Flow ml/min** | **A%** | **B%** |
|---|---|---|---|
| initial | 0.200 | 90.0 | 10.0 |
| 5.00 | 0.200 | 90.0 | 10.0 |
| 30.00 | 0.200 | 0.0 | 100.0 |
| 32.00 | 0.200 | 0.0 | 100.0 |
| 32.01 | 0.200 | 90.0 | 10.0 |
| 35.00 | 0.200 | 90.0 | 10.0 |

According to the UPLC chromatograph results of the enzymatic reaction mixture, the chromatographic peak with retention time of 13±0.5min(the peak position of bradykinin) was integrated and summed, and compared with the peak area of per unit mass bradykinin (1mg/ml, loading volume 10µl) to calculate the concentration of bradykinin generated by the enzymatic reaction. The amount of bradykinin produced was calculated according to the total reaction system in Table 2, and finally converted to the amount of bradykinin(µg/mg) produced per milligram of enzyme at each molar ratio. The results were shown in Table 4.

**Table 4**

| **Molar ratio of substrate to enzyme** | **Bradykinin production µg/mg (enzyme)** | | | |
|---|---|---|---|---|
| | **hKtK1X1** | **PEG10K-hKLK1X1** | **Kb** | **PEG-Kb** |
| **1: 1** | 17.22 | 14.52 | 20.92 | 11.28 |
| **5: 1** | 122.71 | 73.01 | 90.81 | 44.63 |
| **10: 1** | 249.44 | 142.69 | 166.01 | 79.99 |
| **15: 1** | 339.03 | 192.45 | 217.86 | 103.85 |
| **20: 1** | 451.52 | 248.25 | 287.65 | 119.89 |

The detection results based on natural substrates showed that the bradykinin produced by enzymatic reaction of the modified protein was lower than that of the unmodified sample on the whole trend, which was consistent with the characteristics of PEG modified protein. The PEG-modified protein can more modestly maintain the enzymatic process than the original protein, achieving sustained release of effector molecules. At the same time, the effect of KLK1 drugs is based on the regulation of the KKS system in vivo, which includes the release and clearance of effector molecules. Obviously, a mild and continuous enzymatic process can effectively reduce the clearance mechanism of the KKS system, so that the drugs provided by the invention can play their biological effects more stably and effectively.

### Example 2 Activation of downstream signals of the B2 receptor by KLK1

CHO-K1 cells (with B2 receptors) in the logarithmic growth phase were collected and viable cell counts were performed using a hemocyte-counting plate. The live cell suspension was adjusted to 3 × 10⁶cells/mL with medium and seeded in 6-well cell culture plates to a final cell concentration of 3 × 10⁵ cells/ well. The cells were cultured in a CO₂ incubator at 37.0 C and 5.0% CO₂ for 24-48 hours. When the cell density was more than 90%, the cells were washed twice with PBS. The samples were prepared with F12 medium (KLK1 and LMWK were mixed at a mass ratio of 1:10, the reaction system was F12 medium, and the reaction lasted for 15min). After the reaction, 1ml sample was added to each well, and the final concentration of KLK1 was 0.1µM, and the control group was F12 medium. Then the cell culture plate was placed in an incubator at 37.0°C and 5.0% CO₂ for 0.5h. After the incubation, the 6-well plate was removed from the incubator and washed twice with PBS, and the PBS was aspirated after the last wash. The prepared protein lysate (RIPA, PMSF, phosphatase inhibitor) was added at 80µL/well, and the bottom of the well was ground back and back with a scraper to accelerate cell lysis for 1min. The cell debris and the lysate was transferred to a centrifuge tube and centrifuged at 12000rpm for 10min at 4°C. The protein supernatant was carefully aspirated and mixed with 5×Loading Buffer at a volume ratio of 4:1, and then boiled in a metal bath at 100°C for 10min. After cooling at room temperature, the samples were stored at 4°C for short term storage and -20°C for long term storage. The same amount of protein samples and pre-stained markers were loaded into 10% SDS-PAGE for gel running, membrane transfer, blocking, antibody incubation, and luminescence identification.

cAMP-response element binding protein (CREB) was signal molecule after B2R activation. Activation of CREB can prevent inflammatory response and neuronal damage after ischemia, the level of which may reflect the ability of the test substance to activate B2R, and at the same time may reflect the neuroprotective effect.

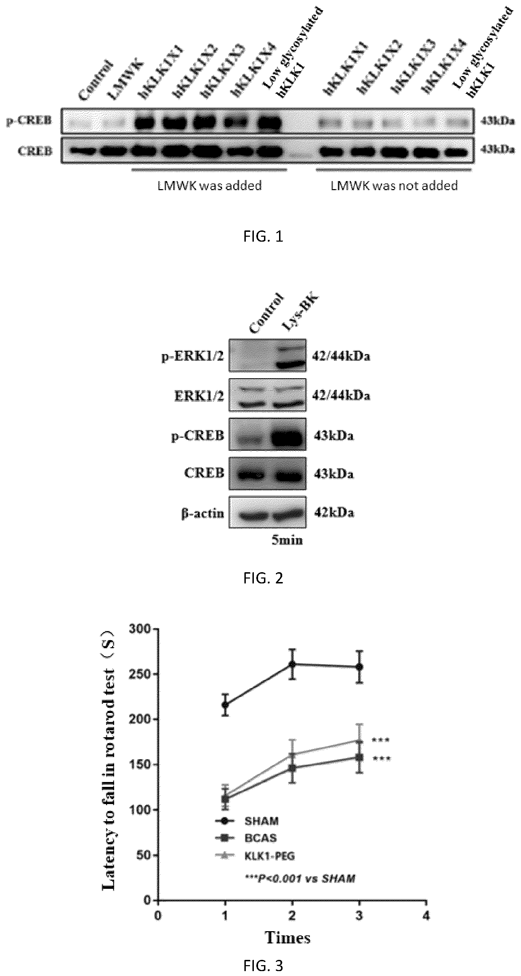

Based on the B2-NFAT-CHO-K1 cell line, it was proved that KLK1 can catalyze LMWK to generate active molecules that binded to the B2 receptor and activated the CREB protein. As shown in Figure 1, KLK1 can activate the phosphorylation of the downstream CREB protein, which is consistent with the action mechanism of the drug. The in vitro biological activity of the samples was qualitatively evaluated by judging the phosphorylation level of the downstream CREB protein. The results showed that hKLK1X1, hKLK1X2, hKLK1X3, hKLK1X4, low glycosylated hKLK1 and high glycosylated hKLK1 all showed the activity of activating downstream pathways after interacting with LMWK.

### Example 3 Activation of downstream signals of the B2 receptor by lysyl bradykinin Lys-BK

CHO-K1 cells (with B2 receptor) in the logarithmic growth phase were collected, and viable cell counts were performed using a hemacyte counting plate. The live cell suspension was adjusted to 3 × 10⁶cells/mL with medium and seeded in 6-well cell culture plates to a final cell concentration of 3 × 10⁵ cells/ well. The cells were cultured in a CO₂ incubator at 37.0°C and 5.0% CO₂ for 24-48 hours. When the cell density was more than 90%, the cells were washed twice with PBS. The sample (Lys-BK) was prepared with F12 medium in advance, and 1ml of sample was added to each well with a final concentration of 1µM, while the control group was F12 medium. Then the cell culture plate was placed in an incubator at 37.0°C and 5.0% CO₂ for 0.5h. Then, the 6-well plate was removed from the incubator and washed twice with PBS, and the PBS was aspirated after the last wash. The prepared protein lysate (RIPA, PMSF, phosphatase inhibitor) was added at 80µL/ well, and the bottom of the well was ground back and back with a scraper to accelerate cell lysis for 1min. The cell debris and the lysate was transferred to a centrifuge tube and centrifuged at 12000rpm for 10min at 4°C. The protein supernatant was carefully aspirated and mixed with 5 × Loading Buffer at a volume ratio of 4:1, and then boiled in a metal bath at 100°C for 10min. After cooling at room temperature, the samples were stored at 4°C for short term storage and -20°C for long term storage. The same amount of protein samples and pre-stained markers were loaded into 10% SDS-PAGE for gel running, membrane transfer, blocking, antibody incubation, and luminescence identification.

Extracellular regulated protein kinases 1/2 (ERK1/2) and cAMP-response element binding protein (CREB) were signal molecule after B2R activation. The activation of CREB and ERK1/2 can prevent the inflammatory response and neuronal damage after ischemia, the level of which may reflect the ability of the test substance to activate B2R, and at the same time may reflect the neuroprotective effect.

Based on the B2-NFAT-CHO-K1 cell line, it was proved that the binding of lysyl bradykinin Lys-BK to B2 receptor activated the downstream signaling CREB and p-ERK1/2. As shown in Figure 2, Lys-BK could activate the phosphorylation of downstream CREB and p-ERK1/2.

### Example 4 Pharmacological effects of KLK1-PEG in mouse BCAS model

### Experiment 1

### I. Grouping and experimental design

Mouse model of bilateral common carotid artery stenosis (BCAS) with cerebral small vessel ischemic injury was established by spring constriction of bilateral common carotid artery.

Preparation of BCAS cerebral small vessel injury model: The BCAS cerebral small vessel ischemic injury model was used to achieve persistent chronic forebrain ischemia in mice. The mice were anesthetized with isoflurane. First, the mice were anesthetized in the induction box of the anesthesia machine, and then the mice were placed supine and connected with a breathing mask. The skin was prepared and sterilized, the neck was cut in the middle, and the bilateral common carotid artery was separated. The bilateral common carotid arteries were constricted with custom-made spring (diameter: 0.08mm, inner diameter: 0.18mm, pitch: about 0.5mm, total length: 2.5mm) to block most of the blood flow, resulting in the decrease of cerebral blood supply and persistent chronic cerebral ischemia. The neck skin was sutured, sterilized, and animals were returned to the cage for feeding.

The experiment was divided into three groups: SHAM operation group (SHAM group), model control group (BCAS group) and KLK1-PEG group.

Drug administration was started on the 3rd day after BCAS surgery, once a week for 7 times. The dose of KLK1-PEG group was 80µg/kg (said KLK1 was hKLK1X1, PEG was SPA10K), the BCAS group and the SHAM group were injected with the same volume of normal saline once a week via the tail vein.

Rotarod test was performed to detect the motor coordination function of mice on the 13th and 26th day after surgery. The mice were placed on the rotarod (30mm diameter ×60mm length) in the opposite direction of rotation, accelerated the rotarod uniformly at 20rpm/min to 40rpm/min and then maintained. The time of mice on the rotarod (that is, the latency to fall) was recorded. Each mouse was tested three to five times with an interval of 20 to 30min. The mean value was taken as the latency to fall, and a longer latency to fall indicated better motor coordination.

Wire hang test was performed to detect the damage of forelimb muscle strength on the 20th and 28th days after surgery. The forelimbs of the mice were suspended on a steel wire (1.5mm diameter ×60cm length), and the time of the mice on the wire (that is, the latency to fall) was recorded. Each mouse was tested 3 to 5 times with an interval of 20 to 30min, and the mean value was taken as the latency to fall. Longer latency to fall indicated less muscle damage.

Y Maze test was performed to detect the impairment of working memory of mice on the 29th and 44th day after surgery. Y maze was a "Y-shaped" experimental device composed of three identical arms 120° away from each other. Each arm was 30cm long, 8cm wide and 15cm high. The test taked advantage of the nature of rodents to explore new environments and was used for researches such as learning and memory. The mice were placed in the Y maze and allowed to move freely. The order of the mice entering each arm with all four paws was recorded for 7min. All arm entries were defined as the number of arm entries, and the number of consecutive entries into all three arms was defined as the number of alternation, with the alternation score as the Y Maze evaluation index. Alternation score = [number of alternation / (number of arm entries -2)]* 100. Animals with less than eight arm entries during the experiment were eliminated (because the data did not reflect precise changes). Higher alternation scores indicated less impaired working memory.

Morris Water Maze Test was performed on D48 to test the spatial learning and memory ability of the mice. The water temperature of the maze was maintained at (23 ± 2)°C, and the pool was divided into four quadrants (E, S, W, N) with four entry points marked on the pool wall. A camera with a display system was placed above the maze to synchronously record the movement trajectory of the mice. The reference objects outside the maze were kept constant during the training period to aid mice positioning. Taking the platform latency (the time from entering the water to finding the platform) as an indicator, the shorter platform latency indicated the milder impairment of spatial learning and memory.

### II. Results

Compared with SHAM group, BCAS group showed significant statistical differences in the results of rotarod test, wire hang test, Y maze test and Morris maze test, indicating that BCAS modeling led to impairment of motor coordination, forelimb muscle strength, working memory and spatial learning and memory. Compared with BCAS model group, KLK1-PEG group had improvement in the above tests. The BCAS model is a more recognized experimental animal model in the study of vascular cognitive impairment and cerebral small vessel disease. The above results indicated that KLK1 or its derivatives can be used for the treatment of cerebral small vessel disease and vascular cognitive impairment.

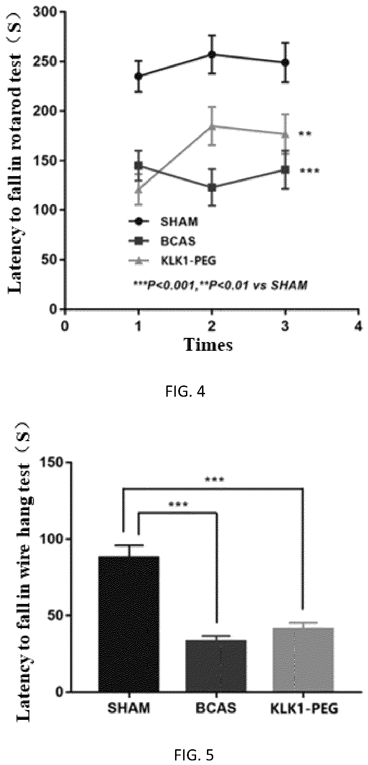

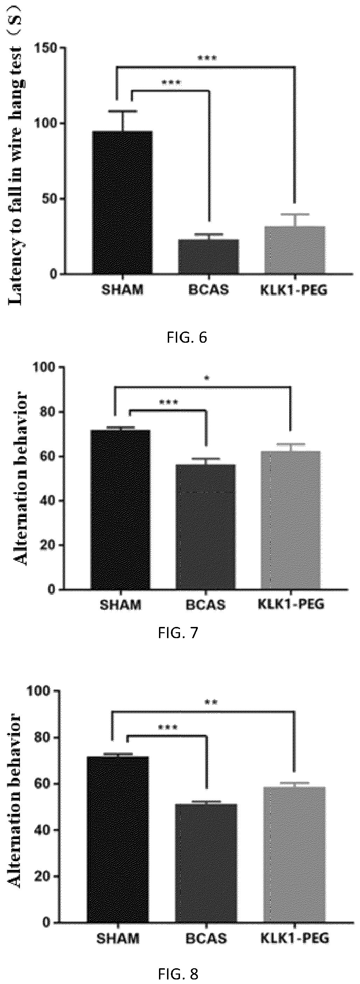

The results were shown in Figures 3-9 and Tables 5-8.

**Table 5 Effect of intravenous administration on latency to fall in rotarod test**

| Number of observations | Groups | Number of animals | Latency to fall(s) on 13d (mean value ± standard error) | Latency to fall(s) on 26d (mean value ± standard error) |
|---|---|---|---|---|
| 1 | SHAM | 16 | 216 ± 11.8 | 235 ± 15.7 |
| | BCAS | 17 | 112 ± 11.5 | 145 ± 15.2 |
| | KLK1-PEG | 16 | 116 ± 11.8 | 121 ± 15.7 |
| 2 | SHAM | 16 | 261 ± 16.4 | 257 ± 19.2 |
| | BCAS | 17 | 146 ± 16.0 | 123 ± 18.6 |
| | KLK1-PEG | 16 | 161 ± 16.4 | 185 ± 19.2 |
| 3 | SHAM | 16 | 258 + 17.6 | 249 ± 19.9 |
| | BCAS | 17 | 158 ± 17.1 | 141 ± 19.3 |
| | KLK1-PEG | 16 | 177 ± 17.6 | 177 ± 19.9 |

**Table 6 Effect of intravenous administration on latency to fall in wire hang test**

| Groups | Number of animals | Latency to fall(s) on 20d (mean value ± standard error) | Latency to fall(s) on 28d (mean value ± standard error) |
|---|---|---|---|
| SHAM | 16 | 88 ± 8.0 | 94 ± 14.1 |
| BCAS | 17 | 33 ± 3.8*** | 22 ± 4.4*** |
| KLK1-PEG | 16 | 41 ± 4.3*** | 31 ± 8.6*** |

| | | | |
|---|---|---|---|
| ***P<0.001, **P<0.01, compared with SHAM group. | | | |

**Table 7 Effect of intravenous administration on alternation behavior in Y Maze test**

| | Groups | Number of animals | Alternation behavior (%) in Y Maze (mean value ± standard error) |
|---|---|---|---|
| 29days | SHAM | 16 | 71.3 ± 1.7 |
| | BCAS | 17 | 55.8 ± 3.2*** |
| | KLK1-PEG | 16 | 61.8 ± 3.7* |
| 44days | SHAM | 16 | 71.2 ± 1.6 |
| | BCAS | 17 | 50.5 ± 1.8*** |
| | KLK1-PEG | 16 | 57.9 ± 2.5** |

| | | | |
|---|---|---|---|
| ***P<0.001, **P<0.01, *P<0.05, compared with SHAM group. | | | |

**Table 8 Effect of intravenous administration on platform latency (s) in Morris maze test**

| Number of times | Groups | Number of animals | Platform Latency (s) (mean value ± standard error) |
|---|---|---|---|
| 1 | SHAM | 12 | 51.7 ± 4.29 |
| | BCAS | 16 | 50.5 ± 2.98 |
| | KLK1-PEG | 14 | 52.0 ± 2.27 |
| 2 | SHAM | 12 | 30.1 ± 4.82 |
| | BCAS | 16 | 49.3 ± 3.07 |
| | KLK1-PEG | 14 | 41.1 ± 4.21 |
| 3 | SHAM | 12 | 29.9 ± 3.79 |
| | BCAS | 16 | 45.0 ± 2.88 |
| | KLK1-PEG | 14 | 38.3 ± 5.00 |
| 4 | SHAM | 12 | 22.5 ± 2.71 |
| | BCAS | 16 | 41.8 ± 3.36 |
| | KLK1-PEG | 14 | 36.0 ± 5.55 |
| 5 | SHAM | 12 | 14.7 ± 2.14 |
| | BCAS | 16 | 37.7 ± 3.44 |
| | KLK1-PEG | 14 | 35.2 ± 3.98 |

### Experiment 2

### I. Grouping and experimental design

Mouse model of bilateral common carotid artery stenosis (BCAS) with cerebral small vessel ischemic injury was established by spring constriction of bilateral common carotid artery.

The BCAS cerebral small vessel injury model was prepared as in experiment I.

The experiment consisted of three groups, namely model group (BCAS group), KLK1 group, and KLK1-PEG group (the KLK1 was hKLK1X1, and the PEG was SPA10K).

Drug administration was started 72 hours after BCAS surgery. The KLK1 group (0.5mg/kg) was injected intravenously 5 times a week, and the KLK1-PEG group (0.1mg/kg) was injected intramuscularly once a week for 8 weeks.

Rotarod test was performed to detect the motor coordination function of mice after 8 weeks of drug administration, and the evaluation method was the same as experiment 1. Wire hang test was performed to detect the damage of forelimb muscle strength of mice after 8 weeks of drug administration, and the evaluation method was the same as experiment 1. Morris maze test was performed to detect the impairment of spatial learning and memory of mice after 8 weeks of drug administration. The water surface was divided into 4 quadrants, and the platform was placed in the center of the III quadrant. In the test phase, the platform in water was removed, and the time and distance of animals moving in different quadrants were recorded. The proportion of time and distance spent in quadrant III to the total time and distance reflected the memory ability of the animal, and the higher the proportion, the less impaired the spatial learning and memory.

### II. Results

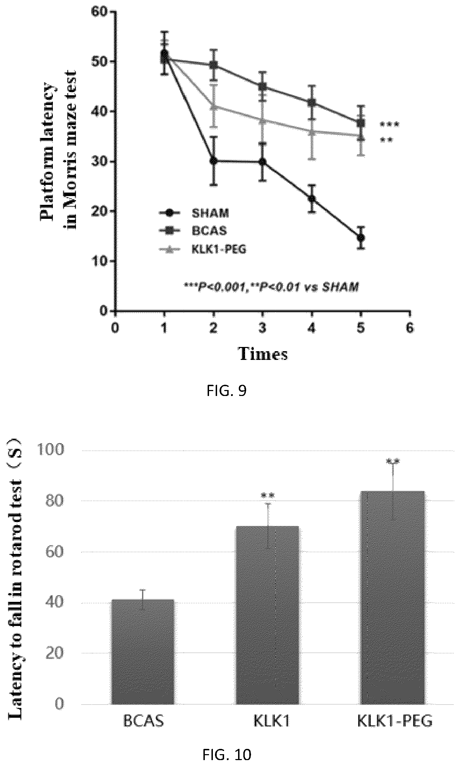

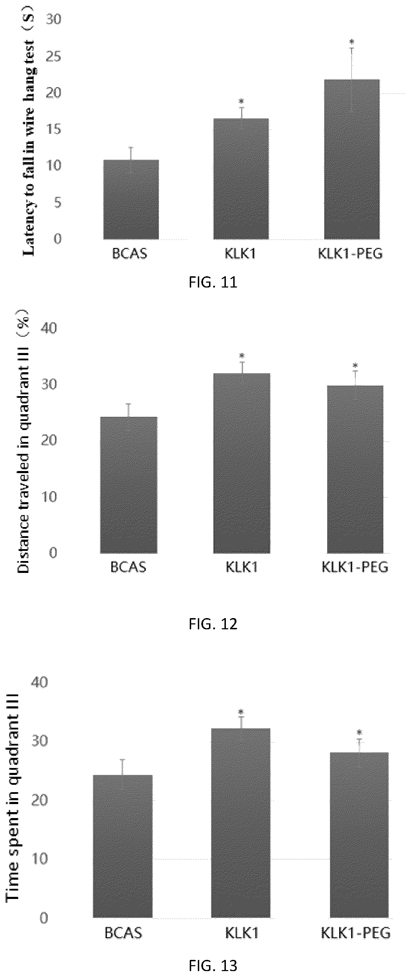

Compared with the model group, the KLK1 group and KLK1-PEG group showed significant statistical differences in the results of rotarod test, wire hang test and Y maze test. KLK1 or KLK1-PEG can significantly improve the motor coordination, forelimb muscle strength, spatial learning and memory impairment caused by BCAS model. It can be used to treat cerebral small vessel disease and vascular cognitive impairment. The results were shown in Figures 10 to 13 and Table 9 to 11.

**Table 9 Effect of intravenous administration on latency to fall(s) in rotarod test**

| Groups | Number of animals | Latency to fall(s) (mean value ± standard error) |
|---|---|---|
| BCAS | 11 | 41.15±3.94 |
| KLK1 | 11 | 70.06±9.05** |
| KLK1-PEG | 11 | 83.66±10.94** |

| | | |
|---|---|---|
| ** indicated P<0.01 compared with the model group. | | |

**Table 10 Effect of intravenous administration on latency to fall(s) in wire hang test**

| Groups | Number of animals | Latency to fall(s) (mean value ± standard error) |
|---|---|---|
| BCAS | 12 | 10.83±1.76 |
| KLK1 | 12 | 16.50±1.46* |
| KLK1-PEG | 10 | 21.80±4.33* |

| | | |
|---|---|---|
| * indicated P<0.05 compared with the model group. | | |

**Table 11 Effect of intravenous administration on platform latency in Morris maze**

| Groups | Number of animals | Distance in quadrant III (%) (mean value ± standard error) | Time spent in quadrant III (%) (mean value ± standard error) |
|---|---|---|---|
| BCAS | 11 | 24.22±2.36 | 24.39±2.54 |
| KLK1 | 16 | 32.08±1.82* | 32.24±1.98* |
| KLK1-PEG | 15 | 29.86±2.51* | 28.08±2.40* |

| | | | |
|---|---|---|---|
| * indicated P<0.05 compared with the model group. | | | |

The embodiment used BCAS model, which was a more recognized experimental animal model in the study of vascular cognitive impairment and cerebral small vessel disease. BCAS model mice treated with KLK1 or polyethylene glycol modified KLK1 can improve the impaired motor coordination function, forelimb muscle strength, working memory, spatial learning and memory function. It can be used to treat vascular cognitive impairment, post-stroke cognitive impairment, and cerebral small vessel disease.

In addition to the test drugs, BCAS mice treated with other KLK1 derivatives that retained the biological function of KLK1 to catalyze LMWK hydrolysis to release lysyl bradykinin, such as other KLK1 mutants (hKLK1X2, hKLK1X3, hKLK1X4, etc.), partial proteins, fusion proteins, other PEG modified product, and other forms of modified product, can be observed to improve the impaired motor coordination function, forelimb muscle strength, working memory, spatial learning and memory function of BCAS model mice. They can be used to treat vascular cognitive impairment, post-stroke cognitive impairment, and cerebral small vessel disease.

## Claims

1. The use of kallikrein I or its derivatives in the preparation of drugs for the treatment or improvement of vascular cognitive impairment.

2. A pharmaceutical composition for the treatment or improvement of vascular cognitive impairment, comprising kallikrein I or a derivative thereof.

3. A method of treating or improving vascular cognitive impairment, in which a patient is given kallikrein I or a derivative thereof.

4. The use of kallikrein I or its derivatives in the preparation of drugs for the treatment or improvement of post-stroke cognitive impairment.

5. A pharmaceutical composition for treating or improving post-stroke cognitive impairment, comprising kallikrein I or a derivative thereof.

6. A method of treating or improving post-stroke cognitive impairment, in which a patient is given kallikrein I or derivative thereof.

7. The use of kallikrein I or its derivatives in the preparation of drugs for the treatment or improvement of Alzheimer's disease with vascular lesions.

8. A pharmaceutical composition for the treatment or improvement of Alzheimer's disease with vascular lesions, comprising kallikrein I or a derivative thereof.

9. A method of treating or improving Alzheimer's disease with vascular lesions, in which the patient is given kallikrein I or a derivative thereof.

10. The use of kallikrein I or its derivatives in the preparation of drugs for the treatment or improvement of cerebral small vessel disease or cognitive dysfunction due to cerebral small vessel disease.

11. A pharmaceutical composition for the treatment or improvement of cerebral small vessel disease or cognitive dysfunction due to cerebral small vessel disease, comprising kallikrein I or a derivative thereof.

12. A method of treating or improving cerebral small vessel disease or cognitive dysfunction due to cerebral small vessel disease, in which kallikrein I or its derivative is administered to a patient.

13. Kallikrein I as described in any one of claim 1 to 12 is native human tissue kallikrein I, mutated or unmutated recombinant human tissue kallikrein I.

14. Kallikrein I derivatives as described in any one of claim 1 to 12 is full-length protein, partial protein or mutant of kallikrein I, fusion protein, various forms of modified product.

15. The kallikrein I derivative of any one of claims 1 to 12 is pegylated kallikrein I.

16. The pegylated kallikrein I of claim 15 is kallikrein I modified with M-SPA-5K, M-SPA-10K, Y-PALD-30K, or Y-PALD-40K.
